# EUROPEAN PATENT SPECIFICATION

(11) **EP 3 960 097 B1**
(45) Date of publication and mention of the grant of the patent: **24.09.2025**
(21) Application number: 20795434.8
(22) Date of filing: 21.04.2020
(51) Int. Cl.: A61B 17/12

(54) **OCCLUDER SUPPORT AND PREPARATION METHOD THEREFOR**
STÜTZE FÜR OKKLUDER UND HERSTELLUNGSVERFAHREN DAFÜR
SUPPORT D'OBTURATEUR ET SON PROCÉDÉ DE PRÉPARATION

(30) Priority: 22.04.2019 CN 201910324887
(43) Date of publication of application: 02.03.2022
(73) Proprietor: Shanghai Microport Cardioadvent Co., Ltd., Shanghai 201203 (CN)
(72) Inventor: ZHOU, Yi, Shanghai 201203 (CN); ZHU, Zexun, Shanghai 201203 (CN); ZHOU, Ting, Shanghai 201203 (CN); CHU, Huimin, Shanghai 201203 (CN); YAO, Yao, Shanghai 201203 (CN); JIANG, Zailai, Shanghai 201203 (CN); LI, Junfei, Shanghai 201203 (CN)
(74) Representative: Patentship Patentanwaltsgesellschaft mbH
(86) International application number: PCT/CN2020/085831
(87) International publication number: WO 2020/216207

(56) References cited:
- CN-A- 104 768 476
- CN-A- 106 175 867
- CN-A- 107 440 760
- CN-A- 108 463 188
- CN-A- 110 522 486
- CN-U- 206 792 446
- CN-U- 207 693 626
- US-A1- 2014 018 841
- US-A1- 2014 052 233
- US-A1- 2017 224 354
- US-A1- 2019 110 880

## Description

### TECHNICAL FIELD

The present invention relates to the technical field of implantable medical instruments and, more specifically, to an occluder support and a preparation method thereof.

### BACKGROUND

Atrial fibrillation (AF) is the most common perpetual arrhythmia seen in clinical practice and its incidence increases with age. In atrial fibrillation, turbulent atrial blood flow can lead to thrombus formation in the left atrial appendage (LAA). Patients with atrial fibrillation suffer from a higher incidence of thrombosis, typically 5-6 times that among healthy people. Therefore, the prevention of atrial fibrillation is of great significance. Statistics to date have shown that it is 90% probable for a patient with non-valvular atrial fibrillation to develop blood clotting in the left atrial appendage. In recent years, studies have shown that LAA occlusion can effectively prevent the risk of ischemic stroke originated from atrial fibrillation.

Existing LAA occlusion devices are generally categorized into two types: internal occluder and external occluder. An internal occluder is shaped like a cage, which is entirely deployed in an LAA and anchored therein with hooks. An external occlude has an umbrella-shaped structure composed of an anchoring mechanism linked to an occlusion disc. In use, the anchoring mechanism is positioned in an LAA to anchor the occluder in place. Although the anchoring mechanism also makes a contribution to occlusion of the LAA, the latter relies mainly on the occlusion disc fitted at the orifice. However, all these existing occluders have been found to be associated with the problem of insufficient safety and manipulability during use.

US 2014/018841 A1 discloses an occlusion device for an atrial appendage.

US 2017/224354 A1 discloses percutaneous medical devices for implantation into the left atrial appendage of a heart.

US 2019/110880 A1 discloses implantable medical devices with anchors.

### SUMMARY OF THE INVENTION

The present invention is set out in the appended set of claims.

It is an objective of the present invention to provide an occluder support and a preparation thereof so as to ensure that the occluder has enhanced manipulability and safety, a wider scope of application and an improved therapeutic effect.

In order to achieve the above objective, the present invention provides an occluder support, having a closed proximal end and an open distal end, the occluder support comprising a mesh body and a trailing structure connected to the mesh body, the mesh body having an inner surface facing a central longitudinal axis of the mesh body, wherein the trailing structure is composed of a plurality of circumferentially arranged struts that are inwardly folded into a blunt shape and then connected to the inner surface of the mesh body.

Optionally, the mesh body is formed integrally with the trailing structure.

Optionally, the trailing structure forms an angle in a range of 90°~180° with a longitudinal axis of the mesh body.

Optionally, the angle between the trailing structure and the longitudinal axis of the mesh body is in a range of 110°~150°.

Optionally, the trailing structure is composed of a plurality of circumferentially arranged struts, each of the struts is connected to the mesh body at one end and has a portion at another end, and the portion is inwardly folded into a blunt shape and connected to the mesh body.

Optionally, the struts are connected to the mesh body detachably or undetachably.

Optionally, the struts are sutured, tied, snapped or riveted to the mesh body.

Optionally, the mesh body comprises several mesh cells having vertices, and wherein each of the struts is connected to the mesh body at a corresponding one of the vertices.

Optionally, the one end of each of the struts is connected to the mesh body and the other end of the strut passes through the mesh body and protrudes out of an outer surface thereof to form an anchoring spike.

Optionally, a support region and an anchoring region is defined in the mesh body in sequence from a proximal end to a distal end, and wherein each of the struts is connected to the anchoring region at the one end and the portion at the other end is connected to the support region or to the anchoring region.

Optionally, the occluder support further comprises a plurality of anchoring spikes secured to the mesh body, wherein at least one connecting hole is formed in the mesh body, and at least one of the anchoring spikes passes through a respective one of the at least one connecting hole to connect with respective one(s) of the struts.

Optionally, the trailing structure is composed of a plurality of mesh cells, each of the mesh cells is connected to the mesh body at one end and has a portion at another end, and the portion is inwardly folded into a blunt shape and connected to the mesh body.

The present invention also provides a preparation method of an occluder support, the occluder support having a closed proximal end and an open distal end, and comprising: cutting a tube to form a mesh body and a trailing structure connected to the mesh body; wherein the mesh body has an inner surface facing a central longitudinal axis of the mesh body, and the trailing structure is composed of a plurality of circumferentially arranged struts; inwardly folding the struts into a blunt shape; and connecting the inwardly folded struts to the inner surface of the mesh body.

Optionally, the trailing structure forms an angle in a range of 90°~180° with a longitudinal axis of the mesh body.

In the occluder support and preparation method thereof according to the present invention, the trailing structure of the occluder support that is inwardly folded into a blunt shape has an increased contact area with an intended organ such as an LAA, which avoids the trailing structure from injuring the LAA wall or the inner tissue of the LAA during and after implantation of the occluder support, thus reducing patient trauma, lowering the risk of pericardial effusion or perforation and making the surgical procedure safer. Moreover, avoiding the trailing structure of the occluder support from injuring LAA tissue during implantation results in enhanced manipulability of the occluder by allowing the surgeon to easily adjust the location where the occluder is implanted in a timely way and hence in reduced surgical complexity and a better therapeutic effect.

Further, the inwardly folded trailing structure allows the occluder support to have a shorter length, making it applicable to a wider range of patients, especially those with a relatively short LAA neck. The inwardly folded trailing structure also imparts increased compressibility and support performance to the occluder support, which ensures good implantation outcomes and even wider applicability to LAAs of various shapes and sizes.

### BRIEF DESCRIPTION OF THE DRAWINGS

Those of ordinary skill in the art would appreciate that the following drawings are presented merely to enable a better understanding of the present invention rather than limit the scope thereof in any sense. In the drawings:
Fig. 1a is a schematic diagram showing the structure of a left atrial appendage (LAA) occluder according to an embodiment of the present invention;
Fig. 1b is a schematic diagram showing a trailing structure of a conventional occlude support that is being implanted into an LAA, where it is possible for the trailing structure to injure LAA when the occlude support is further advanced;
Fig. 1c is a schematic diagram showing a trailing structure of an occlude support of the present invention that is being implanted into an LAA, where the LAA tissue is prevented from being injured by the trailing structure even when the occluder support is further advanced;
Fig. 1d is a schematic diagram showing outermost mesh cells formed by cutting an end portion of a metal tube in accordance with an embodiment of the present invention;
Fig. 2a is an isometric view of an occluder support with a trailing structure employing a unilateral folding design according to an embodiment of the present invention;
Fig. 2b is a front view of an occluder support with a trailing structure employing a unilateral folding design according to an embodiment of the present invention;
Fig. 2c is a partially enlarged view of an occluder support with a trailing structure employing a unilateral folding design according to an embodiment of the present invention;
Fig. 3a is an isometric view of an occluder support with a trailing structure employing a bilateral folding design according to an embodiment of the present invention;
Fig. 3b is a front view of an occluder support with a trailing structure employing a bilateral folding design according to an embodiment of the present invention;
Fig. 3c is a partially enlarged view of an occluder support with a trailing structure employing a bilateral folding design according to an embodiment of the present invention;
Fig. 3d is a schematic view of a trailing structure inclined at an angle A relative to a longitudinal axis of a mesh body according to an embodiment of the pre-sent invention;
Fig. 4a is a schematic partial view of an occluder support with a trailing structure employing a unilateral folding design according to an embodiment of the present invention, showing a connecting hole formed around an anchoring spike;
Fig. 4b is a schematic partial view of an occluder support with a trailing structure employing a bilateral folding design according to an embodiment of the present invention, showing an anchoring spike that is inserted through a connecting hole in the occluder support and connected to the trailing structure; and
Fig. 5 is a schematic view of an LAA occluder assembled with a delivery system according to an embodiment of the present invention.

In these figures,
10 denotes a conventional occlude support; 11, a trailing structure;
100, an LAA occluder;
110, an occluder support; 111, a mesh body; 111a, a proximal region; 111b, a support region; 111c, an anchoring region; 112, a trailing structure; 113, a strut; 114, a connecting hole; 115, an anchoring spike; 116, an outermost mesh cell; 117, a most distal portion of an outermost mesh cell;
120, membrane;
201, a push rod; 202, a delivery sheath tube; and
"A", an angle between a trailing structure and a longitudinal axis of a mesh body.

Like reference numerals across the several views refer to identical or equivalent features.

### DETAILED DESCRIPTION

Objects, advantages and features of the present invention will become more apparent from the following more detailed description of the present invention made in conjunction with the accompanying drawings. Note that the figures are provided in a very simplified form not necessarily drawn to exact scale for the only purpose of helping to explain the disclosed embodiments in a more convenient and clearer way.

As used in the specification and in the appended claims, the singular forms "a", "an" and "the" include plural referents unless the context clearly dictates otherwise. As used in the specification and in the appended claims, the term "or" is employed in the sense including "and/or" unless the context clearly dictates otherwise.

In the following description, the terms "distal", "proximal", "axial" and "circumferential" may be used for the sake of ease of description. When used to de-scribe a delivery device for a left atrial appendage (LAA) occluder, the term "distal" refers to a side away from the device, while the term "proximal" refers to a side close to the device. When used to describe an occluder support in the LAA occluder, the term "axial" refers to a direction along a longitudinal axis of the occlusion occluder support, while the term "circumferential" refers to a direction about the longitudinal axis. Moreover, the term "inwardly" refers to a direction approaching the longitudinal axis of the occluder support, while the term "outwardly" refers to a direction going away from the longitudinal axis. As used herein, the term "plurality" is used in the sense of "two or more" and "several" of "one or more", unless otherwise specified.

The following description sets forth numerous specific details in order to provide a more thorough understanding of the present invention. However, it would be apparent to those skilled in the art that the present invention can be practiced without one or more of these specific details. In other instances, well-known technical features have not been described in order to avoid unnecessary obscuring of the present invention.

The present invention provides an occluder support suitable for use in various lumens or cavities in the body of a mammalian subject, such as blood vessels, the LAA, the heart and the like.

The present invention is described in detail below with reference to the accompanying drawings in the exemplary context of an LAA occluder, and those skilled in the art may apply it to another lumen in the body of a human subject.

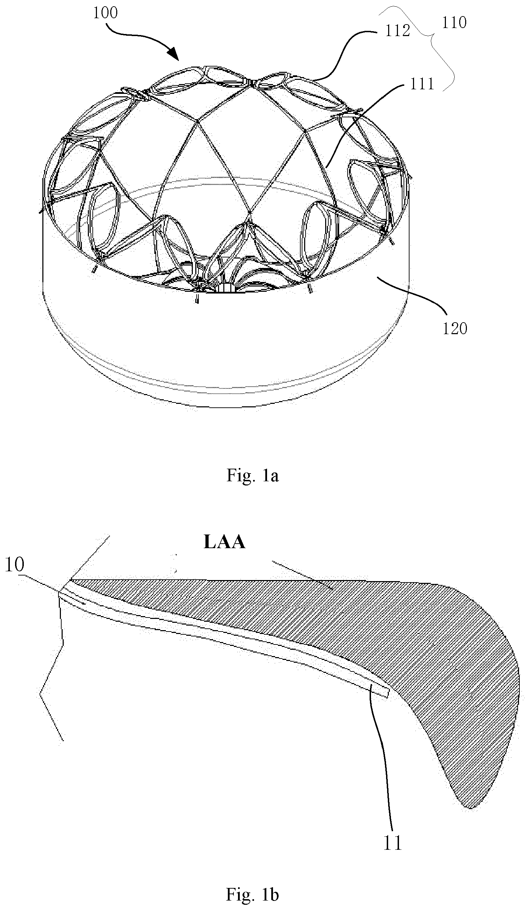

Fig. 1a is a structural diagram showing the structure of an LAA occluder according to an embodiment of the present invention. As shown in Fig. 1a, the LAA occluder 100 includes an occluder support 110. Preferably, the LAA occluder 100 further includes a membrane 120 covering the entire interior or exterior of the occluder support 110 or part thereof. The membrane 120 is designed to effectively block a blood clot from passing through the orifice of an LAA. Preferably, the membrane 120 is formed of a polymeric material.

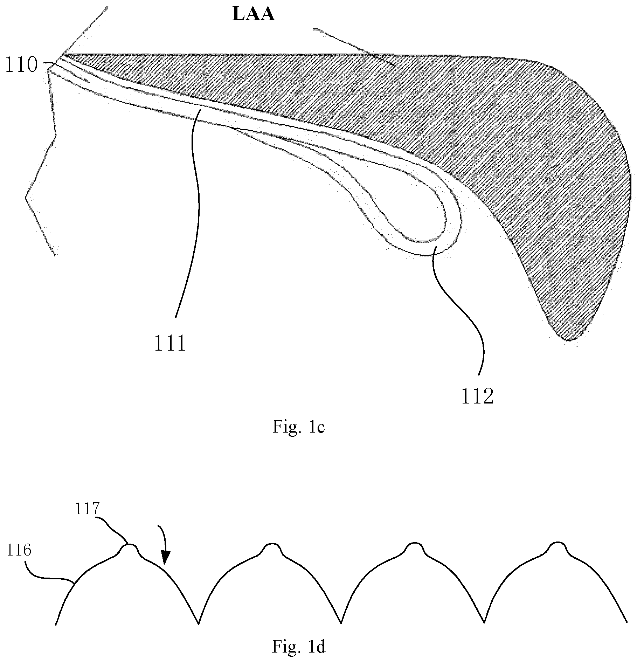

In this embodiment, the LAA occluder 100 is of an internal occluder and is configured to be entirely deployed and held in place with anchors inside the LAA. The LAA occluder 100 is distally open. The occluder support 110 includes a mesh body 111 and a trailing structure 112 connected to the mesh body 111. In particular, the trailing structure 112 is inwardly folded (or bent) into a blunt shape, as can be best seen in Fig. 1c. Here, the term "blunt shape" is intended to mean that the trailing structure 112 continues smoothly from the mesh body 111 so that they together define a non-sharp (blunt) shape such as an arc or a like shape. In the following description, for the sake of brevity, the occluder support is further described by way of preferred embodiments with an "inwardly folded" trailing structure. However, it would be appreciated that the following description is also applicable to embodiments with an "inwardly bent" trailing structure.

Advantages that can be achieved by preferred embodiments of the present invention will be demonstrated in detail by way of a comparison with a conventional occluder support shown in Fig. 1b and an occlude support according to the present application shown in Fig. 1c. For the sake of brevity, components of the conventional occluder support are depicted in a simplified schematic form, or some of them are simply omitted, in Figs. 1b to 1c. However, those skilled in the art would know how such components are implemented based on the teachings disclosed herein and available knowledge in the art.

As shown in Fig. 1b, the conventional occluder support 10 has a trailing end portion 11 with sharp tips, which may easily injure LAA tissue and thus cause pain or even pericardial effusion or perforation to the patient in severe cases during implantation of the conventional occluder support 10. By contrast, as shown in Fig. 1c, according to this embodiment, the blunt (non-sharp) trailing structure 112 of the occluder support 110 will not injure LAA tissue during implantation of the occlude support. This not only makes the surgical procedure safer but makes the occluder easier to manipulate. For instance, when finding that the occluder is positioned externally too much, the surgeon can separately adjust the position of the occluder by pushing it forward, without needing to operate the entire delivery device, resulting in reduced manipulation complexity and a shortened procedure time. Moreover, the inwardly folded trailing structure 112 allows the occluder support 110 to have a shorter length, making it applicable to a wider range of patients, especially those with a relatively short LAA neck. Further, the inwardly folded trailing structure 112 imparts increased compressibility and support performance to the occluder support 110, which ensures good implantation outcomes and even wider applicability to LAAs of various shapes and sizes.

Preferably, the mesh body 111 is formed integrally with the trailing structure 112. This can not only ensure sufficient support that the occluder provides but can also simplify the fabrication process and reduce its cost. Optionally, the occluder support 110 has a self-expanding structure made preferably of an elastic or super elastic material, more preferably of a shape memory elastic material such as a nick-el-titanium alloy. The occluder support 110 may be fabricated by cutting a metal tube. The occluder support 110 fabricated in this way has desirable support strength and good LAA occlusion performance. The cutting is preferably accomplished with a laser.

Further, the trailing structure 112 is composed of a plurality of arranged circumferentially struts 113. Each of these struts 113 is inwardly folded into a blunt shape. In other words, each of the circumferentially arranged struts 113 that make up the trailing structure 112 is connected to the mesh body 111 at one end and has a portion at the other end, which is inwardly folded into a blunt shape (i.e., a section/segment of the strut 113 extending from the other end toward the one end). The blunt shape has a most distal crest point, and at least a portion of the strut 113 between the crest point and the other end (i.e., said portion of the strut 113 at the other end) is connected to the mesh body 111. In some embodiments, the struts 113 may be folded either individually or in pairs. In a preferred embodiment, the occluder support 110 is fabricated by cutting a single metal tube. Specifically, the metal tube may be partially cut so that the uncut portion forms a proximal end portion of the occluder support 110 and the cut portion assumes the form of a mesh network containing several mesh cells and consisting of multiple struts. Each mesh cell includes an opening and has at least three vertices. Preferably, in this embodiment, each mesh cell has four vertices. Mesh cells close to the proximal end define a proximal region, and the others define a distal support region. In one embodiment, the metal tube is so cut that struts 113 each inwardly folded into a blunt shape and connected to one of the outermost ones of the mesh cells are formed at the end opposite to the uncut portion. In another embodiment, as shown in Fig. 1d, among the mesh cells 116 formed in the portion of the metal tube at the opposite end to the uncut portion, the outermost ones of the mesh cells 116 are each inwardly folded at their most distal portions 117 in the direction of the arrow shown in the figure into a blunt shape.

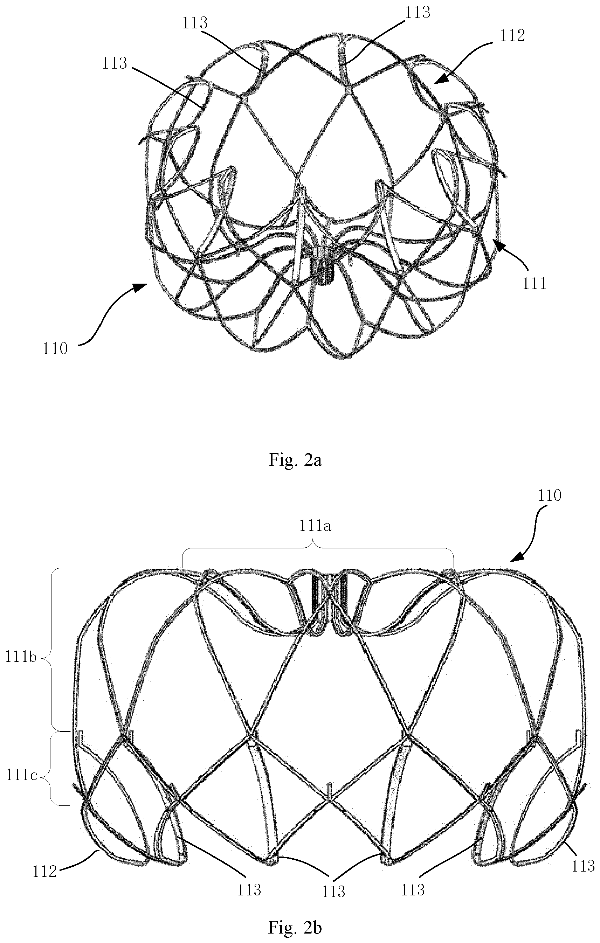

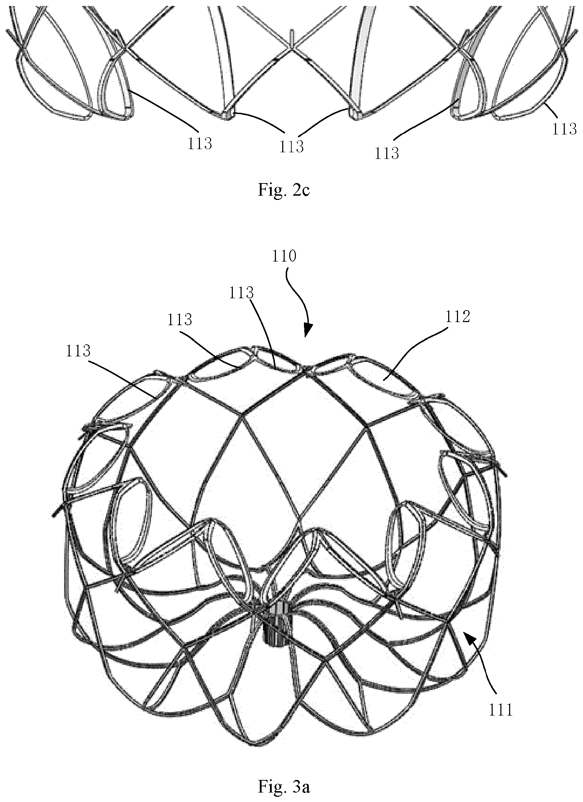

Specifically, the mesh body 111 has a peripheral rim defined by circumferentially arranged crest points. As shown in Figs. 2a to 2c, only one strut 113 leads from each crest point of the peripheral rim and is inwardly folded into a blunt shape. This design is called unilateral folding. Preferably, each strut 113 is folded and then connected to the mesh body 111 in a detachable way, for example, by snapping, suturing, tying or the like, and alternatively in an undetachable way, for example, by welding, riveting, bonding or the like. Coupling the struts 113 to the mesh body 111 imparts to the occluder support increased compressibility and hence enhanced support performance. More preferably, the junctions of the inwardly folded struts 113 with the mesh body 111 are spaced from one another circumferentially, e.g., uniformly on a same circumference. That is to say, the struts 113 are connected to the mesh body 111 at different positions.

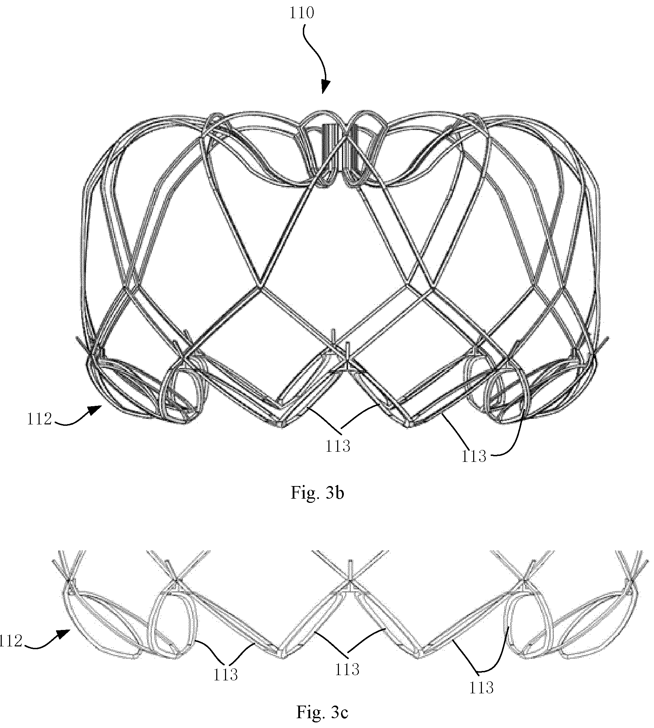

As another example, as shown in Figs. 3a to 3c, two struts 113 lead from each crest point of the peripheral rim and meet with respective two struts 113 from an adjacent crest point. The two pairs of joined struts 113 are then inwardly folded into a blunt shape. Alternatively, the two struts 113 leading from each crest point are both connected to the mesh body. This design is called bilateral folding. The struts 113 are connected to the mesh body in a detachable way, for example, by snapping, suturing, tying or the like, and alternatively in an undetachable way, for example, by welding, riveting, bonding or the like. More preferably, the junctions of the inwardly folded struts 113 with the mesh body 111 are also spaced from one another circumferentially, e.g., uniformly on a same circumference. That is, the struts 113 are connected to the mesh body 111 at different positions.

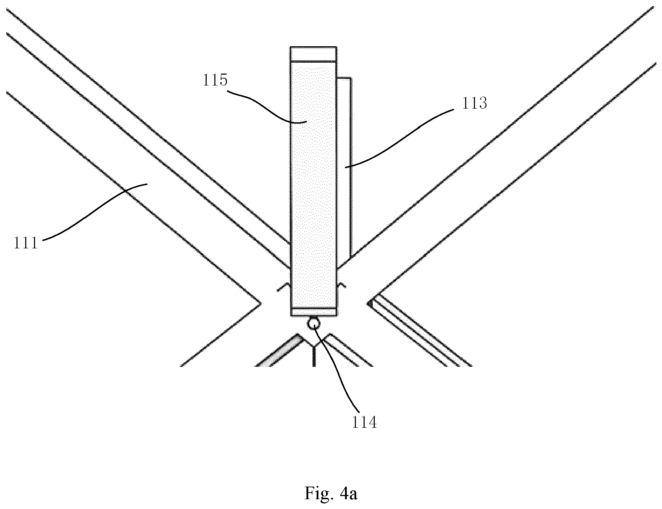

In the unilaterally folding design, as shown in Fig. 4a, a plurality of connecting holes 114 are preferably formed in the mesh body 111, e.g., at vertices or in side struts of some mesh cells. Preferably, the connecting holes 114 are formed at the vertices, i.e., the struts 113 are preferably connected to the mesh body 111 at the vertices, which allows better support performance. Each strut 113 is inserted into a corresponding one of the connecting holes 114 and then fastened with a suture, a wire, a snap, a rivet or the like so that it is connected to the mesh body 111. Preferably, the struts 113 are inserted through the connecting holes 114 so as to protrude out of the external surface of the mesh body 111, and the external protruding portions form anchoring spikes 115. This dispense with separately arranged anchors, resulting in a simpler structure with reduced cost.

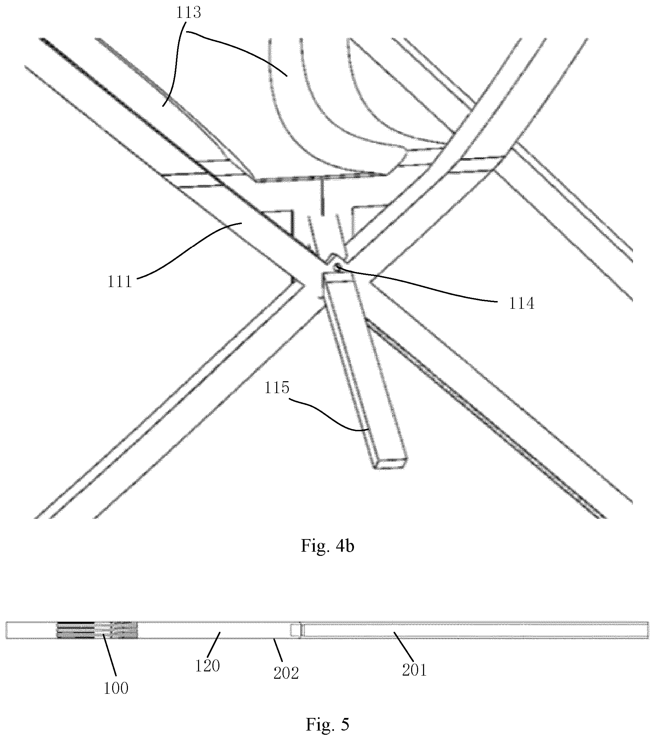

In the bilateral folding design, as shown in Fig. 4b, connecting holes 114 are preferably formed at vertices of some mesh cells in order to enable the coupling of the pairs of joined struts 113 to the mesh body 111 by suturing, snapping, tying or riveting them to the mesh body 111 using the connecting holes 114. Preferably, each pair of joined struts 113 has an extension, which is inserted through a corresponding one of the connecting holes 114 to provide an anchoring spike 115. Alternatively, the two struts 113 in each pair are directly inserted through a corresponding one of the connecting holes 114 to form anchoring spikes 115. Still alternatively, separate anchoring spikes 115 may be provided, which are inserted through connecting holes 114 in the mesh body 111 and connected to the inwardly folded struts 113. The anchoring spikes 115 may be engaged with the connecting holes 114 through interference fit and then fastened with sutures or other wires. Alternatively, snaps or rivets may be used for coupling to ensure that the anchoring spikes 115 will not disengage from the connecting holes 114 during use.

In the above case with the most distal portions 117 of the outermost mesh cells 116 being inwardly folded into a blunt shape, the most distal portions 117 are preferably also connected to the mesh body 111, i.e., each of the mesh cells 116 is connected to the mesh body 111 at one end, and a portion of each of the mesh cells 116 at the aforesaid other end (i.e., a section/segment of the specific mesh cell 116 extending from the other end toward the one end) is inwardly folded into a blunt shape and connected to the mesh body 111. The coupling may be accomplished in a similar manner as described above in connection with the unilateral or bilateral folding design and is therefore not described again.

The present invention is not limited to any particular number of anchoring spikes 115, and these anchoring spikes are fixed to the mesh body 111 and configured to anchor the occluder to an LAA.

Further, a proximal region, a support region and an anchoring region are defined in the mesh body 111 in sequence from a proximal end to a distal end. The trailing structure 112 is connected to the anchoring region, and the proximal region is configured to be connected to a delivery device. The support region is configured to provide sufficient support. The anchoring spikes are provided in the anchoring region for anchoring. For example, in case of the occluder support 110 adopting the unilateral folding design, the mesh body 111 may has a proximal region 111a, a support region 111b and an anchoring region 111c, as shown in Fig. 2b. Here, the regions are defined by their functions, and each of them is not limited to any particular range. The anchoring region 111c may be considered as corresponding to a portion of the occluder support extending from the roots of the anchoring spikes 115 upward over 10%~30% of a total length of the occluder support.

The proximal region 111a is provided with a proximal securing member (not labeled) configured for coupling to the delivery device. The proximal region 111a is inwardly recessed to provide the occluder support 110 with some support and resilience, which ensures that the occluder will regain its original shape after being released from a sheath tube. The support region 111b is composed of several supporting mesh cells and may extend in parallel, or at a certain angle with respect, to the longitudinal axis of the mesh body 111. In the former case, better support can be provided. The anchoring region 111c is outwardly bulged in order to provide some support and enable the anchoring spikes 115 to penetrate tissue of the LAA to prevent dislodgement therefrom.

In some embodiments, the inwardly folded struts 113 may be selectively connected to any one of the proximal region 111a, the support region 111b and the anchoring region 111c. Preferably, they are connected to the support region 111b or the anchoring region 111c to facilitate entry or exit of the occluder into or from the sheath tube. However, the region to which the struts are connected should be determined according to the actual strength requirements to form the blunt shaped trailing structure.

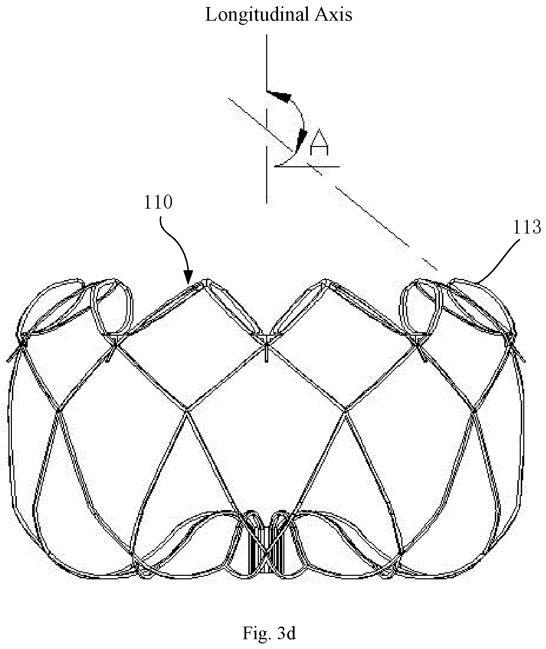

Additionally, in embodiments of the present invention, in order to achieve improved support performance of the occluder support, the trailing structure 112 is preferably inclined at an angle of 90°~180° relative to the longitudinal axis of the mesh body. Here, if a positive direction of the longitudinal axis is defined as going from the proximal to distal end of the occluder support, then with respect to this positive direction, each strut 113 is bent at an angle in the range from 90° to 180°, such as for example, 90°, 100°, 110°, 125°, 135°, 160° or 180°. More preferably, an angle A between the trailing structure 112 and the longitudinal axis of the mesh body 11 ranges from 110° to 150°. The angle A is defined, for each strut 113, as an angle between the longitudinal axis and a line connecting the original point of the strut with the farthest point of the strut away from the original point, as shown in Fig. 3d. The angle A in the aforesaid range can effectively enhance the ability of the occluder support to anchor inside the LAA, thus ensuring good reliability of the occluder during use. Further, the angle A may be selected from the range of 110°~120°, 115°~125°, 120°~130°, 125°~135°, 130°~140°, 135°~145° or 140°~150°.

With continued reference to Fig. 1a, the membrane 120 may cover the mesh body 111 from the proximal region 111a up to the anchoring region 111c. This not only allows thrombus occlusion over a wider range and hence increased implantation process tolerance, but can also avoid direct contact of the metal occluder support with tissue and thus facilitate endothelialization.

As shown in Fig. 5, in embodiments of the present invention, there is also provided a delivery device for delivering the LAA occluder 100 into the body of a patient. Specifically, the delivery device includes a push rod 201 having a distal end for detachable coupling to the proximal securing member of the occluder support 110. In this way, delivery and deploy of the LAA occluder can be achieved with the push rod 201. The delivery device further includes a sheath tube 202 configured to load the LAA occluder 100 and guide it into the body of the patient. The push rod 201 may be threadedly connected to the proximal securing member. Such a threaded coupling is not only reliable but can also be easily established and removed.

According to some embodiments, the push rod 201 may be either solid or hollow. In the latter case, it may be a flexible tube in which, for example, a guide wire, a suture, an endoscope or a detectable device may be transferred to address various surgical needs.

In summary, according to embodiments of the present invention, the trailing structure of the occluder support is inwardly folded into a blunt shape, thereby resulting in an increased contact area with an LAA, which avoids the trailing structure from injuring the LAA wall or surrounding tissue during and after implantation of the occluder support, thus reducing patient trauma, lowering the risk of pericardial effusion or perforation and making the surgical procedure safer. Moreover, avoiding the trailing structure of the occluder support from injuring LAA tissue during implantation results in enhanced manipulability of the occluder by allowing the surgeon to easily adjust the location where the occluder is implanted in a timely way and hence in reduced surgical complexity and a better therapeutic effect.

Further, the inwardly folded trailing structure allows the occluder support to have a shorter length, making it applicable to a wider range of patients, especially those with a relatively short LAA neck. The inwardly folded trailing structure also imparts increased compressibility and support performance to the occluder support, which ensures good implantation outcomes and even wider applicability to LAAs of various shapes and sizes.

The description presented above is merely that of a few preferred embodiments of the present invention and is not intended to limit the scope thereof in any sense.

## Claims

1. An occluder support (110), having a closed proximal end and an open distal end, the occluder support (110) comprising a mesh body (111) and a trailing structure (112) connected to the mesh body (111), the mesh body (111) having an inner surface facing a central longitudinal axis of the mesh body (111), wherein the trailing structure (112) is composed of a plurality of circumferentially arranged struts (113) that are inwardly folded into a blunt shape and
**characterised in that** the trailing structure (112) is then connected to the inner surface of the mesh body (111).

2. The occluder support (110) of claim 1, wherein the mesh body (111) is formed integrally with the trailing structure (112).

3. The occluder support (110) of claim 1 or 2, wherein the trailing structure (112) forms an angle (A) in a range of 90°~180° with respect to a positive direction of a longitudinal axis of the mesh body (111).

4. The occluder support (110) of claim 3, wherein the angle (A) is in a range of 110°~150°.

5. The occluder support (110) of claim 1, wherein the struts (113) are connected to the mesh body (111) detachably or undetachably.

6. The occluder support (110) of claim 5, wherein the struts (113) are sutured, tied, snapped or riveted to the mesh body (111).

7. The occluder support (110) of claim 1, wherein the mesh body (111) comprises several mesh cells having vertices, and wherein each of the struts (113) is connected to the mesh body (111) at a corresponding one of the vertices.

8. The occluder support (110) of claim 1, wherein the one end of each of the struts (113) is connected to the mesh body (111), and the other end of the strut (113) extends out of the mesh body (111) therethrough to form an anchoring spike (115).

9. The occluder support (110) of any one of claims 1, 5 to 8, wherein a proximal region (111a), a support region (111b) and an anchoring region (111c) are defined in the mesh body (111) in sequence from a proximal end to a distal end, and wherein each of the struts (113) is connected to the anchoring region (111c) at the one end and the portion at the other end is connected to the support region (111b) or to the anchoring region (111c).

10. The occluder support (110) of any one of claims 1, 5 to 8, further comprising a plurality of anchoring spikes (115) secured to the mesh body (111), wherein at least one connecting hole (114) is formed in the mesh body (111), and at least one of the anchoring spikes (115) passes through a respective one of the at least one connecting hole (114) to connect with respective one(s) of the struts (113).

11. The occluder support (110) of claim 1, wherein the trailing structure (112) is composed of a plurality of mesh cells formed by struts (113), each of the mesh cells is connected to the mesh body (111) at one end and has a portion at another end, and the portion is inwardly folded into a blunt shape and connected to the mesh body (111).

12. A preparation method of an occluder support (110), the occluder support (110) having a closed proximal end and an open distal end, the method comprising:
cutting a tube to form a mesh body (111) and a trailing structure (112) connected to the mesh body (111), wherein the mesh body (111) has an inner surface facing a central longitudinal axis of the mesh body (111), and the trailing structure (112) is composed of a plurality of circumferentially arranged struts (113);
inwardly folding the struts (113) into a blunt shape; and
**characterised in that** the method comprises further:
connecting the inwardly folded struts (113) to the inner surface of the mesh body (111).

13. The preparation method of claim 12, wherein the trailing structure (112) forms an angle (A) in a range of 90°~180° with respect to a positive direction of a longitudinal axis of the mesh body (111).

## Patentansprüche

1. Eine Stütze für Okkluder (110) mit einem geschlossenen proximalen Ende und einem offenen distalen Ende, wobei die Stütze für Okkluder (110) einen Netzkörper (111) und eine mit dem Netzkörper (111) verbundene nachlaufende Struktur (112) umfasst, wobei der Netzkörper (111) eine Innenfläche aufweist, die einer zentralen Längsachse des Netzkörpers (111) zugewandt ist, wobei die nachlaufende Struktur (112) aus einer Vielzahl von umlaufend angeordneten Streben (113) besteht, die nach innen in eine stumpfe Form gefaltet sind und **dadurch gekennzeichnet sind, dass** die nachlaufende Struktur (112) dann mit der Innenfläche des Netzkörpers (111) verbunden ist.

2. Die Stütze für Okkluder (110) nach Anspruch 1, wobei der Netzkörper (111) einstückig mit der nachlaufenden Struktur (112) ausgebildet ist.

3. Die Stütze für Okkluder (110) nach Anspruch 1 oder 2, wobei die nachlaufende Struktur (112) einen Winkel (A) in einem Bereich von 90° bis 180° in Bezug auf eine positive Richtung einer Längsachse des Netzkörpers (111) bildet.

4. Die Stütze für Okkluder (110) nach Anspruch 3, wobei der Winkel (A) in einem Bereich von 110° bis 150° liegt.

5. Die Stütze für Okkluder (110) nach Anspruch 1, wobei die Streben (113) lösbar oder unlösbar mit dem Netzkörper (111) verbunden sind.

6. Die Stütze für Okkluder (110) nach Anspruch 5, wobei die Streben (113) an den Netzkörper (111) genäht, gebunden, eingeschnappt oder genietet sind.

7. Die Stütze für Okkluder (110) nach Anspruch 1, wobei der Netzkörper (111) mehrere Netzzellen mit Scheitelpunkten umfasst und wobei jede der Streben (113) an einem entsprechenden Scheitelpunkt mit dem Netzkörper (111) verbunden ist.

8. Die Stütze für Okkluder (110) nach Anspruch 1, wobei das eine Ende jeder der Streben (113) mit dem Netzkörper (111) verbunden ist, und das andere Ende der Strebe (113) durch den Netzkörper (111) hindurchragt, um einen Verankerungsdorn (115) zu bilden.

9. Die Stütze für Okkluder (110) nach einem der Ansprüche 1, 5 bis 8, wobei in dem Netzkörper (111) nacheinander von einem proximalen Ende zu einem distalen Ende ein proximaler Bereich (111a), ein Stützbereich (111b) und ein Verankerungsbereich (111c) definiert sind und wobei jede der Streben (113) an dem einen Ende mit dem Verankerungsbereich (111c) verbunden ist und der Abschnitt an dem anderen Ende mit dem Stützbereich (111b) oder dem Verankerungsbereich (111c) verbunden ist.

10. Die Stütze für Okkluder (110) nach einem der Ansprüche 1, 5 bis 8, die ferner eine Vielzahl von Verankerungsdornen (115) umfasst, die an dem Netzkörper (111) befestigt sind, wobei mindestens ein Verbindungsloch (114) in dem Netzkörper (111) ausgebildet ist und mindestens einer der Verankerungsdorne (115) durch ein jeweiliges der mindestens einen Verbindungslöcher (114) verläuft, um mit der oder den jeweiligen Strebe(n) (113) verbunden zu werden.

11. Die Stütze für Okkluder (110) nach Anspruch 1, wobei die nachlaufende Struktur (112) aus einer Vielzahl von durch Streben (113) gebildeten Netzzellen besteht, wobei jede der Netzzellen an einem Ende mit dem Netzkörper (111) verbunden ist und am anderen Ende einen Abschnitt aufweist, und der Abschnitt nach innen in eine stumpfe Form gefaltet und mit dem Netzkörper (111) verbunden ist.

12. Ein Herstellungsverfahren für eine Stütze für Okkluder (110), wobei die Stütze für Okkluder (110) ein geschlossenes proximales Ende und ein offenes distales Ende aufweist, wobei das Verfahren umfasst:
Schneiden eines Rohrs, um einen Netzkörper (111) und eine mit dem Netzkörper (111) verbundene nachlaufende Struktur (112) zu bilden, wobei der Netzkörper (111) eine Innenfläche aufweist, die einer zentralen Längsachse des Netzkörpers (111) zugewandt ist, und die nachlaufende Struktur (112) aus einer Vielzahl von umlaufend angeordneten Streben (113) besteht;
Falten der Streben (113) nach innen in eine stumpfe Form; und
**dadurch gekennzeichnet, dass** das Verfahren weiterhin umfasst:
Verbinden der nach innen gefalteten Streben (113) mit der Innenfläche des Netzkörpers (111).

13. Das Herstellungsverfahren nach Anspruch 12, wobei die nachlaufende Struktur (112) einen Winkel (A) in einem Bereich von 90° bis 180° in Bezug auf eine positive Richtung einer Längsachse des Netzkörpers (111) bildet.

## Revendications

1. Support d'obturateur (110) comportant une extrémité proximale fermée et une extrémité distale ouverte, le support d'obturateur (110) comprenant un corps en maille (111) et une structure arrière (112) reliée au corps en maille (111), le corps en maille (111) ayant une surface intérieure faisant face à un axe longitudinal central du corps en maille (111), dans lequel la structure traînante (112) est composée d'une pluralité d'entretoises disposées circonférentiellement (113) qui sont pliés vers l'intérieur dans une forme émoussée et **caractérisés en ce que** la structure arrière (112) est ensuite connectée à la surface intérieure du corps en maille (111).

2. Support d'obturateur (110) selon la revendication 1, dans lequel le corps en maille (111) est formé d'un seul tenant avec la structure arrière (112).

3. Support d'obturateur (110) selon la revendication 1 ou 2, dans lequel la structure arrière (112) forme un angle (A) dans une plage de 90° à 180° par rapport à une direction positive d'un axe longitudinal du corps en maille (111).

4. Support d'obturateur (110) selon la revendication 3, dans lequel l'angle (A) est compris dans une plage de 110° à 150°.

5. Support d'obturateur (110) selon la revendication 1, dans lequel les entretoises (113) sont reliées au corps en maille (111) de manière détachable ou non détachable.

6. Support d'obturateur (110) selon la revendication 5, dans lequel les entretoises (113) sont suturées, attachées, enclenchées ou rivetées au corps en maille (111).

7. Support d'obturateur (110) selon la revendication 1, dans lequel le corps de maille (111) comprend plusieurs cellules de maille ayant des sommets, et dans lequel chacune des entretoises (113) est connectée au corps de maille (111) au niveau d'un sommet correspondant parmi les sommets.

8. Support d'obturateur (110) selon la revendication 1, dans lequel une extrémité de chacune des entretoises (113) est reliée au corps en maille (111), et l'autre extrémité de la jambe de force (113) s'étend hors du corps en maille (111) pour former une pointe d'ancrage (115).

9. Support d'obturateur (110) selon l'une quelconque des revendications 1, 5 à 8, dans lequel une région proximale (111a), une région de support (111b) et une région d'ancrage ( 111c) sont définies dans le corps de maille (111) en séquence d'une extrémité proximale à une extrémité distale, et dans lequel chacune des entretoises (113) est connectée à la région d'ancrage (111c) à une extrémité et la partie à l'autre extrémité est connectée à la région de support (111b) ou à la région d'ancrage (111c).

10. Support d'obturateur (110) selon l'une quelconque des revendications 1, 5 à 8, comprenant en outre une pluralité de pointes d'ancrage (115) fixées au corps en treillis (111), dans lequel au moins un trou de connexion (114) est formé dans le corps en maille (111), et au moins une des pointes d'ancrage (115) passe à travers un trou respectif parmi les au moins un trou de connexion (114) pour se connecter avec l'une ou les entretoises respectives (113).

11. Support d'obturateur (110) selon la revendication 1, dans lequel la structure arrière (112) est composée d'une pluralité de cellules de maille formées par des
entretoises (113), chacune des cellules de maille est connectée au corps de maille (111) à une extrémité et comporte une partie à une autre extrémité, et la partie est pliée vers l'intérieur dans une forme émoussée et connectée au corps de maille (111).

12. Procédé de préparation d'un support d'obturateur (110), le support d'obturateur (110) ayant une extrémité proximale fermée et une extrémité distale ouverte, le procédé comprenant :
couper un tube pour former un corps en maille (111) et une structure de fuite (112) reliée au corps en maille (111), le corps en maille (111) ayant une surface intérieure faisant face à un axe longitudinal central du corps en maille (111), et la structure de fuite (112) étant composée d'une pluralité d'entretoises disposées de manière circonférentielle (113) ;
replier vers l'intérieur les entretoises (113) en une forme émoussée ; et
**caractérisé en ce que** le procédé comprend en outre :
reliant les entretoises pliées vers l'intérieur (113) à la surface intérieure du corps en maille (111).

13. Procédé de préparation selon la revendication 12, dans lequel la structure arrière (112) forme un angle (A) dans une plage de 90° à 180° par rapport à une direction positive d'un axe longitudinal du corps en maille (111).
